# EUROPEAN PATENT SPECIFICATION

(11) **EP 1 535 626 B1**
(45) Date of publication and mention of the grant of the patent: **20.04.2011**
(21) Application number: 05000534.7
(22) Date of filing: 07.06.1996
(51) Int. Cl.: A61K 39/02, A61K 39/114

(54) **Actinomyces pyogenes and actinomyces pyogenes/fusobacterium necrophorum vaccines for immunizing cattle and sheep**
Actinomyces Pyogenes und actinomyces Pyogenes/Fusobakterium Necrophorum Impfstoffe zur Immunisierung von Vieh und Schafen
Vaccins d'actinomyces pyogènes et d'actinomyces pyogènes/fusobactérium nécrophorum utiles pour immuniser le bétail bovin et ovin

(30) Priority: 07.06.1995 US 477619; 07.06.1995 US 483382
(43) Date of publication of application: 01.06.2005
(62) Divisional of application: 96921496.4
(73) Proprietor: Kansas State University Research Foundation, Manhattan, KS 66502 (US)
(72) Inventor: Chengappa, Muckatira M., Manhattan, KS 66503 (US); Nagaraja, Tiruvoor G., Manhattan, KS 66503-8663 (US)
(74) Representative: UEXKÜLL & STOLBERG

(56) References cited:
- WO-A-94/00556
- TAKEUCHI S ET AL: "PATHOGENIC SYNERGISM OF FUSOBACTERIUM NECROPHORUM AND OTHER BACTERIA IN FORMATION OF LIVER ABSCESS IN BALB/C MICE" PROCEEDINGS JPN. SYMPOSIUM PLASMA CHEM, TOKYO, JP, vol. 45, no. 6, 1983, pages 775-781, XP002941942
- SCANLAN C M ET AL: "BOVINE RUMENITIS - LIVER ABSCESS COMPLEX: A BACTERIOLOGICAL REVIEW" CORNELL VETERINARIAN, CORNELL VETERINARIAN, ITHACA, NY, US, vol. 73, 1983, pages 288-297, XP002943850 ISSN: 0010-8901
- DATABASE EMBASE [Online] ELSEVIER SCIENCE PUBLISHERS, AMSTERDAM, NL; 1982, COLLINS M D ET AL: "Reclassification of Corynebacterium pyogenes (Glage) in the genus Actinomyces, as Actinomyces pyogenes comb. nov." XP002323779 Database accession no. EMB-1982159645 & JOURNAL OF GENERAL MICROBIOLOGY 1982 UNITED KINGDOM, vol. 128, no. 4, 1982, pages 901-903,
- BERG J N ET AL: "Fusobacterium necrophorum and Bacteroides melaninogenicus as etiologic agents of foot rot in cattle" AMERICAN JOURNAL OF VETERINARY RESEARCH, vol. 36, no. 8, August 1975 (1975-08), pages 1115-1122, XP002088079 ISSN: 0002-9645
- CAMERON C.M.; FULS W.J.P.: 'Failure to induce in rabbits effective immunity to a mixed infection of Fusobacterium necrophorum and Corynebacterium pyogenes with a combined bacterin' ONDERSTEPOORT J. VET. RES. vol. 44, no. 4, 01 January 1977, ONDERSTEPOORT, SA, pages 253 - 256, XP002941940
- KATITCH R.: 'Les problemes de l`etologie et de l`immunoprophylaxie dans le pietin du mouton' COMP. IMMUN. MICROBIOL. INFECT. DIS. vol. 2, 1979, pages 55 - 59
- CAMERON C.M.; BOTHA W.F.; SMIT B.H.: 'Antibody Response to and Immunity induced by Corynebacterium Pyogenes Vaccine' ONDERSTEPOORT J. VET. RES. vol. 43, no. 3, 01 September 1976, ONDERSTEPOORT, SA, pages 97 - 103, XP009096834

## Description

### Background of the Invention

### 1. Field of the Invention

The present invention is broadly concerned with novel inocula for administration to cattle or sheep in order to lessen or prevent the incidence of liver abscesses and/or foot rot. More particularly, the invention pertains to such inocula, methods of preparing the same and methods of lessening the incidence of liver abscesses and/or foot rot in cattle or sheep via administration of the inocula. The invention relates to a multivalent inoculum comprising a first bacterial component in the form of an inactivated cell culture product of *A. pyogenes* and a second bacterial component in the form of an inactivated cell culture product of *F*. *necrophorum.*

### 2. Description of the Prior Art

Liver abscesses in feed lot cattle are a serious economic problem, causing condemnation of over 3 million livers and an estimated loss of $15 million annually in the United States. This estimate is based primarily on condemnation of liver and other organs, and does not include economic losses stemming from reduced feed efficiencies and lowered weight gains. A number of studies have confirmed that cattle with abscessed livers gain less (average 4-5%) and have reduced feed efficiencies (average 7%) compared with cattle having healthy livers. The average incidence of abscessed liver in grain-fed cattle approximates 25-30%.

Liver abscesses in cattle are part of a disease complex where the abscessation is secondary to primary foci of infection in the rumen epithelium. The pathogenesis can be summarized as follows: (1) ruminal lesions are induced by acidosis that follows rapid change in diet from high-roughage to high grain, prolonged feeding of high grain diet (sometimes referred to as an all-concentrate diet), or occasionally by foreign body penetration of the rumen epithelium; (2) bacteria present in the rumen invade the epithelium and form focal abscesses in the rumen wall; and (3) bacteria enter the portal circulation, and are carried to the liver where they localize in the parenchyma with subsequent abscess formation.

*F. necrophorum* is the primary etiologic agent of liver abscesses in ruminant animals. The organism has been recognized as an animal and human pathogen since the late 1800s, and is associated with numerous necrotic disease conditions in domestic and wild animals. In addition to liver abscesses, the organism is also the primary etiologic agent of foot rot, foot abscesses, calf diphtheria, and is frequently isolated from cases of mastitis, metritis, and necrotic lesions of the oral cavity.

The ability of *F. necrophorum* to establish in the liver is attributed to the production of a toxin called leukotoxin (or leucocidin). The toxin is soluble, proteinaceous and has specificity for bovine leukocytes. The leukotoxin is believed to aid in the establishment of *F. necrophorum* in the liver by directly impairing the normal defense mechanism and indirectly by the damage caused by cytolytic products released from neutrophils and macrophages to the hepatic cells. Therefore, the leukotoxin elaborated from *F. necrophorum* plays a critical role in *F. necrophorum* infection of the liver.

*F. necrophorum* is a gram-negative, nonsporeforming, nonmotile, strictly anaerobic and pleomorphic organism. Morphologically, the organism varies from short rods to filamentous with pointed and rounded ends. Cell lengths range from coracoid bodies of 0.5-0.7 µm in diameter to filaments over 100 µm. Surface colonies are 1-2 mm in diameter, circular, transparent to opaque, and with some strains producing α or β hemolysis. The organism ferments glucose, fructose and maltose only weakly with final pH around 5.0-6.3. It ferments lactate to acetate, propionate, and butyrate. Butyrate is the major product from lactate fermentation. Indole is produced from peptone. *F. necrophorum* has been isolated from the normal flora in the oral cavity, gastrointestinal cavity, and genitourinary tract of humans and animals. The organism is also known to survive in the soil.

Four biotypes (A, B, AB and C) of *F. necrophorum* have been described. Biotype A, most frequently isolated from liver abscesses, is more pathogenic than biotype B, which predominates in ruminal wall abscesses. Biotype AB is rarely isolated, and has pathogenicity intermediate that of biotypes A and B. Biotype C is non-pathogenic.

It has been suggested in the past to utilize *F. necrophorum* bacterin as an agent for immunizing cattle and sheep against liver necrosis, EPO Application No. 460480 of December 11, 1991. Specifically, virulent *F. necrophorum* isolates are inactivated using β-propiolactone, followed by addition of adjuvants. In addition, Abe et al. (Infection and Immunity, 13:1473-1478, 1976) grew *F. necrophorum* for 48 hours. Cells were obtained by centrifuging, washing three times with saline, and were inactivated with formalin (0.4% in saline). The inactivated cells were then injected into mice to induce immunity. Two weeks after the last booster injection, each mouse was challenged with viable cells of *F. necrophorum.* The mice immunized with killed cells and challenged with live cells had no detectable bacteria in the liver, lung or spleen for up to 28 days. It was concluded that immunization of mice with formalin-killed *F. necrophorum* conferred protection against infection. Garcia et al. (Canadian J. Comp. Med, 38:222-226, 1974) conducted field trials to evaluate the efficacy of alum-precipitated toxoids of *F. necrophorum.* The vaccine preparation consisted of washed cells (unlikely to contain leukotoxin) that were ruptured by sonication. The most promising result was achieved with the injection of 15.5 mg protein of cytoplasmic toxoid. In this group, the incidents of liver abscesses was reduced to 10% from an average 35% in the control group. Finally, Emery et al., (Vet. Microbiol., 12:255-268, 1986) prepared material by gel filtration of 18-hour culture supernate of *F. necrophorum.* This elicited significant immunity against challenged from viable *F. necrophorum.* The injected preparation contained endotoxin and the majority of the leukotoxic activity.

PCT Publication WO 94/00556 published January 6, 1994 describes novel *F. necrophorum* leukotoxid vaccines as well as methods of enhancing the elaboration of leukotoxin from *F. necrophorum* and of producing a leukotoxin vaccine. In particular, this publication discloses that *F. necrophorum* bacteria are advantageously cultured in growth media at a temperature of from about 35-41 °C and a pH of from about 6.5-8 for a period of from about 4-10 hours in order to maximize the production of leukotoxin. Thereafter, culturing is terminated and the leukotoxin-bearing supernate is separated and inactivated to form a vaccine.

*Actinomyces pyogenes* is a gram-positive, cocco-bacillary shaped, facultative organism that is associated with a number of pyogenic conditions (termed "Pyobacillosis") in animals and humans. It is frequently isolated in mixed culture with other bacteria including *F. necrophorum.* In liver abscesses and foot rot in cattle, *A. pyogenes* is the second most frequent pathogen isolated. In addition to liver abscesses and foot rot, the organism is also a frequent isolated from pyogenic infections of a number of organs such as lungs, mammary glands, joints and the uterus. the pathogenic mechanism of *A. pyogenes* is not well understood. Some of the factors that contribute to pathogenicity include exotoxins (hemolysin or leukotoxin) and enzymes such as proteinase, DNases, and neuraminidase. There is also evidence that *A. pyogenes* in cattle synergistically interacts with other bacteria including *F. necrophorum* and the combination may be more virulent than individual species. However, no *A. pyogenes*-derived vaccines or inocula for immunizing cattle and sheep against liver abscesses and/or foot rot have been prepared in the past

Takeuchi et al., Proceedings Jpn. Symposium Plasma Chem, Tokyo, JP, 45(6):775-781 (1983) and Scanlan et al., Cornell Veterinarian, Ithaca, NY, US, 73:288-297 (1983) identify C. *pyogenes* as one of pathogens in ruminal lesions and abscesses caused by *F. necrophorum.*

Cameron et al. J. Vet Res. 44(4):253-256 (1977) describes that a vaccine comprising *F*. *necrophorum* and C. *pyogenes* bacterin failed to induce effective immunity in rabbits infected with these pathogens.

Katitch, Comp. Immun. MicroBiol. Infect. Dis. 2:55-59 (1979) reports studies of sheep vaccination against foot rot in Serbia using mono- and multi-valent vaccines based on *Ristella nodosa, Sphaerophorus necrophorus, Staphylococcus pyogenes* and *Clostridium pefringens A.*

### Summary of the Invention

The present invention relates to inocula for cattle and sheep in order to immunize such ruminants and lessen the incidence therein of liver abscesses and/or foot rot. In addition, the invention pertains to methods of preparing such inocula and of lessening the incidence of the aforementioned pathogenic conditions in cattle and sheep.

The invention pertains to a multi-valent inoculum for administration to cattle or sheep in order to lessen the incidence of liver abscesses and/or foot rot therein, said inoculum consisting essentially of first and second bacterial components in a compatible carrier, said first bacterial component consisting essentially of an inactivated cell culture product of *Actinomyces pyogenes,* and said second bacterial component consisting essentially of an inactivated leukotoxin-containing supernatant elaborated by *Fusobacterium necrophorum* cells in cell culture.

The first A. pyogenes-derived bacterial compound is prepared by forming a cell culture of a strain of *A. pyogenes* in a growth media such as Brain Heart Infusion broth or RPMI 1640 media using an overnight culture with incubation at 35-39°C for 12-48 hours. At the end of the incubation period (most preferably after about 36 hours), a cell culture product is inactivated using formalin, ß-propiolactone, heat, radiation or any other known method of inactivation. In particularly preferred forms, the entire cell culture is inactivated and chilled for two days. Thereafter, the inactivated cell culture is centrifuged and the supernatant antigenic material is recovered. As indicated previously, in alternate procedures, the entire cell culture, separate cells or cellular subunits may be used as the antigenic material. In any case, after inactivation and separation as desired, the antigenic material is mixed with a suitable adjuvant carrier.

The second *F. necrophorum-*derived bacterial component is prepared in the manner described in PCT Publication No. WO 94/00556.

In more detail, the *F*. *necrophorum* second bacterial component for the multi-valent vaccine is in the form of inactivated leukotoxin-containing supernatant elaborated by *F. necrophorum* cells in cell culture. Culturing of *F. necrophorum* preferably involves culturing a biotype A strain in a suitable growth medium such as Brain Heart Infusion broth at a temperature of from about 35-41 °C and a pH of from about 6.5-8 for a period of from about 4-9 hours. The preferred strain of *F*. *necrophorum* has ATCC Accession No. 55329. At the end of the culturing step, leukotoxin supernatant is separated and inactivated by any known means, preferably through the use of formalin. This inactivated antigenic material can then be mixed with an appropriate adjuvant, of the same type described with reference to the *A. pyogenes* vaccine preparation.

The final multivalent inoculum in accordance with the invention is prepared by mixing the first and second bacterial components derived from the respective first and second cell cultures. Preferably, the bacterial components are present in the final multivalent inoculum at about a 1:1 (v/v) ratio.

It has been determined that the inocula of the present invention will have particular utility in those cases where *A. pyogenes* becomes a significant etiological agent. For example, it has unexpectedly been found that where cattle or sheep are regularly treated (e.g., fed) with an antibiotic which reduces the incidence of liver abscesses and/or foot rot, a high percentage of infected animals exhibit *A. pyogenes* in their abscess bacterial flora. Accordingly, where ruminant animals are subjected to a regular regime of antibiotics such as tylosin, chlortetracycline, oxytetracycline, bacitracin and mixtures thereof to reduce the incidence of liver abscesses and/or foot rot, these animals should also be treated with the inocula of the present invention. In this way, the incidence of liver abscesses and/or foot rot will be further lessened.

In addition, it is known that cattle fed a high concentrate diet including at least about 90% by weight grain characteristically have a high incidence of liver abscesses (greater than 50%). It has now been discovered that a high percentage of abscessed livers from cattle fed concentrate diets exhibit *A. pyogenes* upon bacteriological examination. Therefore, cattle fed concentrate diets can also materially benefit from treatment with the inocula of the invention.

### Detailed Description of the Preferred Embodiment

The following examples describe the preferred techniques for the production and use of a multivalent inoculum containing *A. pyogenes* and *F. necrophorum* antigenic components. It is to be understood, however, that these examples are presented by way of illustration only.

### Example 1

In this example, the bacterial flora of liver abscesses from feedlot cattle fed with or without the antibiotic tylosin were examined. A total of 36 liver abscesses from tylosin-fed cattle were examined, along with 41 liver abscesses from cattle not fed tylosin. This study revealed that there was a higher incidence of *A. pyogenes* in the liver abscesses of tylosin-fed animals. Accordingly, it was deduced that in tylosin-fed animals, the role of *F. necrophorum* is lessened and *A. pyogenes* assumes a greater role than in animals not fed tylosin. The results of this study are set forth in the following table.

**Table 1. Bacterial flora of liver abscesses in cattle fed tylosin or no tylosin**

| Bacteria | Tylosin | No Tylosin |
|---|---|---|
| No. of abscesses cultured | 36 | 41 |
| *F. necrophorum* | 36/36 (100)^{a} | 41/41 (100) |
| Subsp. *necrophorum^{b}* (biotype A) | 18/36(50) | 34/41 (82) |
| Subsp. *funduliforme* (biotype B) | 3/36 | 1/41 |
| *Actinomyces pyogenes^{b}* | 19/36(53) | 4/41 (100 |
| ^{a}Numbers in parentheses are percentages | | |
| ^{b}Chi Square test P < .01 | | |

Although the origin of *F. necrophorum* found in liver abscesses is well known, the source of *A. pyogenes* is not understood. Because *A. pyogenes* is aerobic, it is not a normal inhabitant of the rumen; however, the bacterium may be a component of the epimural flora of the rumen (bacteria attached to the rumen wall). Facultative or aerobic bacteria have a better chance of surviving in anaerobic conditions by adhering to the rumen wall where extensive blood supply provides oxygen. The close proximity of the rumen wall enhances the opportunity of the bacterium to get into portal circulation and thereby enter the liver. The bacteria could remain dormant and multiply if conditions (such as entry into the liver) become conducive. The higher incidence of *A. pyogenes* in tylosin-fed cattle is surprising because the organism is quite sensitive to tylosin.

### Example 2

In this example, the liver abscesses of cattle fed an all-concentrate diet (100% grain) were bacteriologically examined. It is known that feedlots making use of all-concentrate diets have a high incidence of liver abscesses (greater than 50%). In this study, the livers of 24 animals were examined and it was found that 19 were abscessed. Eighteen of these abscesses were cultured and *F. necrophorum* was found in 12 abscesses whereas *A. pyogenes* was found in 17 abscesses. This data is summarized in the following Table 2.

**Table 2. Bacterial flora of liver abscesses in cattle fed all concentrated diet**

| Bacteria | Incidence | Percentage |
|---|---|---|
| No. of animals | 24 | |
| Abscessed | 19 | 79 |
| No. of abscesses cultured | 18 | |
| *F. necrophorum* | 12 | 67 |
| *Actinomyces pyogenes* | 17 | 94 |

In the basis of the tests sets forth in Examples 1 and 2, it is believed that there is a synergistic interaction between *F. necrophorum* and *A. pyogenes* in causing liver abscesses. The presence of *A. pyogenes* may enhance the virulence of *F. necrophorum* or vice-versa. Table 3 sets forth the characteristics of the respective bacteria and their interactions.

**Table 3.**

| Characteristics | *Fusobacterium necrophorum* | *Actinomyces pyogenes* | Interaction |
|---|---|---|---|
| O₂ relationship | Anaerobic | Facultative | *A*. *pyogenes* utilizes O₂ to create anaerobic condition for *F. necrophorum* growth |
| Substrate and endproducts | Ferments lactate | Produces lactate | *A. pyogenes* provides substrate for *F. necrophorum* growth |
| Leukotoxin production | Strongly positive | Negative or weakly positive | Leukotoxin of *F. necrophorum* protects against phagocytosis |
| Hemolysis | Weakly positive | Strongly positive | Hemolytic activity of *A*. *pyogenes* provides iron required for *F. necrophorum* growth |

### Example 3

In this example, the preparation of a monovalent *A. pyogenes* vaccine is described for the sake of comparision only, as well as the preparation of a multivalent *A*. *pyogenes*/*F. necrophorum* vaccine.

### A. pyogenes Vaccine

A suitable strain of *A. pyogenes* is grown in a Brain Heart Infusion broth (DIFCO Laboratories, Detroit, MI) or RPMI 1640 medium (GIBCO Laboratories, Grand Island, NY). The growth medium is inoculated with an overnight culture (1-5% inoculum size) of *A. pyogenes* and is incubated at 35-39°C for 12-48 hours in a 5% CO₂ atmosphere (a standard atmosphere could also be used). At the end of the incubation period, the culture is inactivated by adding formalin (0.3-0.4%) on a vol./vol. basis. The inactivated whole culture is chilled in an ice bath and refrigerated for one or two days. Thereafter, the inactivated cell culture is centrifuged (13,500 g for 15-30 minutes) and the supernatant antigenic material is recovered. In alternative procedures, the whole cell culture (bacterin) or cellular subunits can be used as the antigenic material.

The antigenic material is then mixed with a suitable adjuvant (e.g., aluminum hydroxide or other commercially available oil-based or metallic salt adjuvant) to complete the vaccine. The vaccine may then be conventionally administered to sheep or cattle (one or more vaccinations) to elicit antibodies in the animals and will prevent the establishment of *A. pyogenes* in the animal's liver or feet, or any other organs.

### A. pyogenes/F. necrophorum Vaccine

The *F. necrophorum* leukotoxoid vaccine component is prepared by growing a *F. necrophorum* biotype A strain (e.g., strain 25, ATCC Accession No. 55329) in a pre-reduced (0.05% cysteine HCl) anaerobically sterilized Brain-Heart Infusion broth (DIFCO Laboratories, Detroit, MI) at 39°C for 7 hours (absorbance 0.8 at 600 nm). The culture supernatant is obtained by centrifugation at 13,500 g for 30 minutes at 4°C, filtered through a 0.45 µm membrane filter (Micron Separations, Inc., Westborough, MA) and inactivated with 0.3% formalin. *F. necrophorum* leukotoxin and protein concentrations in the cell culture supernatant are assayed before and after inactivation with formalin.

The inactivated supernatant is mixed with Ribi adjuvant (premixed, sterile form from Ribi Immunochem Research, Inc., Hamilton, MT) or other adjuvant as described above at a level of 90% leukotoxin supernatant/10% adjuvant. The mixture is then emulsified in a homogenizer.

In order to create the multivalent vaccine, the *A. pyogenes* vaccine and the *F. necrophorum* vaccine component are mixed on a 1:1 v/v basis. The multivalent vaccine can then be conventionally administered (typically via one or more injections) to cattle or sheep.

The multivalent vaccines of the invention may be conventionally administrated via parenteral injection or other known techniques in order to elicit the production of appropriate antibodies in the ruminant animals. Such administrations may be at one time or multiple administrations spaced over a period of time.

## Claims

1. An inoculum for administration to cattle or sheep in order to lessen the incidence of liver abscesses and/or foot rot therein, said inoculum consisting essentially of first and second bacterial components in a compatible carrier, said first bacterial component consisting essentially of an inactivated cell culture product of *Actinomyces pyogenes,* and said second bacterial component consisting essentially of an inactivated leukotoxin-containing supernatant elaborated by *Fusobacterium necrophorum* cells in cell culture.

2. The inoculum of claim 1, said cell culture product of *Actinomyces pyogenes* being selected from the group consisting of separated *Actinomyces pyogenes* cells, *Actinomyces pyogenes* cellular subunits, the supernatant elaborated by *Actinomyces pyogenes* cells, and mixtures thereof.

3. The inoculum of claim 2, said cell culture product being the supernatant elaborated by *Actinomyces pyogenes* cells in cell culture.

4. The inoculum of claim 1, said strain of *Fusobacterium necrophorum* bacteria being a biotype A strain.

5. The inoculum of claim 4, said *Fusobacterium necrophorum* strain having ATCC Accession No. 55329.

6. The inoculum of claim 1, said first and second bacterial components being present in said inoculum at about a 1:1 (v/v) ratio.

7. The inoculum of claim 1, including an adjuvant.

8. The inoculum of claim 7, said adjuvant being taken from the group consisting of aluminium hydroxide and oil-based or metallic salt adjuvants.

9. Use of the inoculum of claims 1 to 8 for the preparation of a pharmaceutical composition for lessening the incidence of liver abscesses in cattle or sheep.

10. Use of the inoculum of claims 1 to 8 for the preparation of a pharmaceutical composition for lessening the incidence of foot rot in cattle or sheep.

## Patentansprüche

1. Ein Inokulum zur Verabreichung an Rinder oder Schafe, um bei diesen die Inzidenz von Leberabszessen und/oder Moderhinke zu verringern, wobei das Inokulum im Wesentlichen aus ersten und zweiten bakteriellen Komponenten in einem kompatiblen Träger besteht, wobei die erste bakterielle Komponente im Wesentlichen aus einem inaktivierten Zellkulturprodukt von *Actinomyces pyogenes* besteht, und wobei die zweite bakterielle Komponente im Wesentlichen aus einem inaktivierten Leukotoxin-enthaltenden Überstand besteht, welcher durch *Fusobacterium necrophorum* Zellen in Zellkultur entwickelt wurde.

2. Das Inokulum nach Anspruch 1, **dadurch gekennzeichnet, dass** das Zellkulturprodukt von *Actinomyces pyogenes* ausgewählt ist aus der Gruppe bestehend aus getrennten *Actinomyces pyogenes* Zellen, *Actinomyces pyogenes* zellulären Untereinheiten, dem durch *Actinomyces pyogenes -* Zellen entwickelten Überstand und Mischungen davon.

3. Das Inokulum nach Anspruch 2, **dadurch gekennzeichnet, dass** das Zellkulturprodukt der von *Actinomyces pyogenes* in Zellkultur entwickelte Überstand ist.

4. Das Inokulum nach Anspruch 1, **dadurch gekennzeichnet, dass** der Stamm von *Fusobacterium necrophorum -* Bakterien ein Biotyp A Stamm ist.

5. Da Inokulum nach Anspruch 4, **dadurch gekennzeichnet, dass** der *Fusobacterium necrophorum* Stamm ATCC Zugangsnummer 55329 hat.

6. Das Inokulum nach Anspruch 1, **dadurch gekennzeichnet, dass** die ersten und zweiten bakteriellen Komponenten in dem Inokulum in etwa einem 1:1 (v/v) Verhältnis anwesend sind.

7. Das Inokulum nach Anspruch 1, **dadurch gekennzeichnet, dass** es ein Adjuvans einschliesst.

8. Das Inokulum nach Anspruch 7, **dadurch gekennzeichnet, dass** das Adjuvans aus der Gruppe genommen wird, welche aus Aluminiumhydroxid und Öl-basierten oder Metallsalz-Adjuvantien besteht.

9. Verwendung des Inokulums nach Ansprüchen 1-8 zur Herstellung einer pharmazeutischen Zusammensetzung zur Verringerung der Inzidenz von Leberabszessen bei Rindern oder Schafen.

10. Verwendung des Inokulums nach Ansprüchen 1-8 zur Herstellung einer pharmazeutischen Zusammensetzung zur Verringerung der Inzidenz von Moderhinke bei Rindern oder Schafen.

## Revendications

1. Inoculum conçu pour être administré à du bétail ovin ou bovin afin de faire baisser l'incidence du piétin et des abcès du foie au sein de ce bétail, lequel inoculum est essentiellement constitué d'un premier composant bactérien et d'un deuxième composant bactérien qui sont mis dans un véhicule compatible, ledit premier composant bactérien étant essentiellement constitué d'un produit inactivé de culture cellulaire *d'Actinomyces pyogenes,* et ledit deuxième composant bactérien étant essentiellement constitué d'un surnageant élaboré par des cellules de *Fusobacterium necrophorum* en culture cellulaire, contenant de la leucotoxine et inactivé.

2. Inoculum conforme à la revendication 1, pour lequel ledit produit de culture cellulaire d'*Actinomyces pyogenes* est choisi dans l'ensemble formé par des cellules séparées *d'Actinomyces pyogenes,* des sous-unités cellulaires *d'Actinomyces pyogenes,* un surnageant élaboré par des cellules *d'Actinomyces pyogenes,* et les mélanges de tels produits.

3. Inoculum conforme à la revendication 2, dans lequel ledit produit de culture cellulaire est un surnageant élaboré par des cellules d'*Actinomyces pyogenes* en culture cellulaire.

4. Inoculum conforme à la revendication 1, pour lequel la souche de bactéries *Fusobacterium necrophorum* est une souche du biotype A.

5. Inoculum conforme à la revendication 4, pour lequel la souche de *Fusobacterium necrophorum* est la souche portant le numéro d'accès ATCC 55329.

6. Inoculum conforme à la revendication 1, dans lequel lesdits premier et deuxième composants bactériens se trouvent présents en un rapport d'environ 1/1 en volume.

7. Inoculum conforme à la revendication 1, qui contient un adjuvant.

8. Inoculum conforme à la revendication 7, pour lequel l'adjuvant est choisi dans l'ensemble formé par l'hydroxyde d'aluminium et les adjuvants à base d'huile ou de type sel métallique.

9. Utilisation d'un inoculum conforme à l'une des revendications 1 à 8 en vue de la préparation d'une composition pharmaceutique conçue pour faire baisser l'incidence des abcès du foie chez du bétail ovin ou bovin.

10. Utilisation d'un inoculum conforme à l'une des revendications 1 à 8 en vue de la préparation d'une composition pharmaceutique conçue pour faire baisser l'incidence du piétin chez du bétail ovin ou bovin.
